# FASCICULE DE BREVET EUROPEEN

(11) **EP 2 240 772 B1**
(45) Date de publication et mention de la délivrance du brevet: **07.09.2016**
(21) Numéro de dépôt: 08857644.2
(22) Date de dépôt: 27.11.2008
(51) Int. Cl.: G01N 33/543

(54) **BIOPUCES FONCTIONNALISÉES POUR LE COUPLAGE SPR-MS**
FUNKTIONALISIERTE BIOCHIPS FÜR SPR-MS-KOPPELUNG
FUNCTIONALISED BIOCHIPS FOR SPR-MS COUPLING

(30) Priorité: 27.11.2007 FR 0759351
(43) Date de publication de la demande: 20.10.2010
(73) Titulaire: Genoptics, 91400 Orsay (FR); Centre National de la Recherche Scientifique, 75016 Paris (FR); Universite d'Evry Val d'Essonne, 91000 Evry (FR)
(72) Inventeur: DANIEL, Régis, F-92130 Issy Les Moulineaux (FR); GONNET, Florence, F-91700 Villiers Sur Orge (FR); BUCHMANN, William, F-91700 Ste Genevieve Des Bois (FR); BELLON, Sophie, F-75012 Paris (FR); JARROUX, Nathalie, F-91160 Ballainvilliers (FR); ANGER-LEROY, Marielle, F-91940 Les Ulis (FR)
(74) Mandataire: Sekhri, Redha
(86) Numéro de dépôt international: PCT/FR2008/052149
(87) Numéro de publication internationale: WO 2009/071852

(56) Documents cités:
- WO-A-03/005890
- WO-A-2006/073465
- LU H B ET AL.: "Attachment of functionalized poly(ethylene glycol) films to gold surfaces" LANGMUIR, ACS, vol. 16, no. 4, 22 février 2000 (2000-02-22), pages 1711-1718, XP002360360 On line: 14 janvier 2000 (2000-01-14)
- BECKER C F W ET AL.: "Functional immobilization of the small GTPase Rab6A on DNA-gold nanoparticles by using a site-specifically attached poly(ethylene glycol) linker and thiol place-exchange reaction" CHEMBIOCHEM, vol. 8, no. 1, 2 janvier 2007 (2007-01-02), pages 32-36, XP009105104 doi:10.1002/cbic.200600422 ISSN: 1439-4227
- MANNELLI I ET AL.: "Bioadhesive nanoareas in antifouling matrix for highly efficient affinity sensors" PROCEEDINGS OF SPIE - THE INTERNATIONAL SOCIETY FOR OPTICAL ENGINEERING - BIOSENSING 2008 SPIE, vol. 7035, 29 août 2008 (2008-08-29), pages 70350Y-1-70350Y-10, XP002529609 US DOI:10.1117/12.796969

## Description

L'invention concerne un procédé permettant de coupler en ligne l'analyse des interactions moléculaires par résonance de plasmons de surface (SPR) à une identification structurale par spectrométrie de masse, en utilisant un même support fonctionnalisé pour les deux types d'analyse.

Avec le développement des sciences protéomiques et dans le prolongement de celui de la génomique, une demande très forte est apparue de technologies analytiques efficaces et à haut débit, notamment pour la recherche et l'identification de biomarqueurs. En outre, l'ensemble des données produites actuellement par l'étude du génome, du protéome et du glycome fait émerger un schéma du fonctionnement cellulaire reposant sur un réseau complexe de biomolécules en interaction dynamique. De nombreux travaux sont réalisés pour tenter de mieux comprendre les différents phénomènes impliqués dans la régulation du protéome. Or, les solutions techniques adéquates permettant l'exploration de ce réseau sont peu nombreuses. Les récentes avancées en protéomique ont été obtenues en faisant évoluer les technologies existantes et en développant de nouvelles approches basées sur le couplage de techniques séparatives avec la spectrométrie de masse. Ainsi est apparue la combinaison de l'électrophorèse 2D avec la spectrométrie de masse, puis le couplage de la chromatographie liquide (LC) avec la spectrométrie de masse LC-MS et LC-MS-MS pour l'identification et l'analyse des protéines.

Plus récemment l'utilisation de biopuces (lab-on-chip : laboratoire sur puce) s'est développée pour l'exploration du génome et du protéome. Concernant l'étude des interactions entre biomolécules, une nouvelle approche s'avère prometteuse en couplant deux techniques analytiques -la Résonance de Plasmons de Surface (Surface Plasmon Resonance ou SPR), et la spectrométrie de masse (Mass Spectrometry ou MS) et plus particulièrement la spectrométrie de masse MALDI avec comme unique interface la biopuce en elle-même. La SPR permet de quantifier et d'analyser du point de vue cinétique et thermodynamique les interactions entre biomolécules, alors que la MS renseigne sur leurs structures moléculaires. Ainsi les premiers résultats de cette stratégie nommée BIA/MS (pour Biomolecular Interaction Analysis /Mass Spectrometry) ont été obtenus en 1997 par l'équipe de Randall W. Nelson. Depuis, plusieurs équipes ont concentré leurs efforts pour faciliter le couplage SPR/MS selon différentes stratégies (*Nedelkov*, *2003*)*.*

La voie principale actuelle consiste en la microélution des molécules retenues sur la puce SPR par des tampons adéquats. Le matériel est ensuite concentré et ses sels échangés sur des résines, éventuellement traité (dérivation, digestion peptidique) avant d'être analysé par spectrométrie de masse. Bien que ces approches par microélution permettent la récupération des molécules retenues en SPR, puis leur analyse par spectrométrie de masse, elles souffrent d'un certain nombre de limitations, notamment la nécessité avant l'analyse MS de plusieurs étapes consommatrices de temps et de réactifs (en effet, le matériel récupéré est souvent élué dans un tampon dénaturant incompatible avec la MS et qu'il faut échanger par un autre tampon). Ces différentes étapes peuvent conduire à des contaminations ainsi qu'à une perte de matériel avec pour conséquences de faibles rendements et des problèmes de détection.

Une seconde stratégie est actuellement en cours d'exploration selon laquelle les deux analyses SPR et MALDI-MS sont effectuées sur la même puce. La plupart de ces travaux de couplage SPR-MS ont été réalisés sur des instruments Biacore, le système d'analyse SPR actuellement le plus répandu. Ce système Biacore emploie une configuration de puce fermée avec une circulation microfluidique interne. Pour une analyse SPR-MS, la puce, après analyse SPR, est extraite du biocapteur, lavée et séchée. Etant située à l'intérieur de la biopuce, la zone d'interaction n'est pas directement accessible et il est donc nécessaire d'ouvrir la biopuce. Cette opération est irréversible et les puces Biacore sont détruites lors de la récupération de la zone d'interaction, et ne peuvent donc pas être ré-utilisées pour des analyses SPR répétées (Nedelkov, J. Mol. Recogn. 2000, 13, 140-145*).* En outre, ce système impose une étape de traitements post-SPR comportant une phase de lavage pour éliminer les détergents ou tampons utilisés pendant l'analyse SPR mais aussi tous les composés présents dans le système fluidique qui peuvent provoquer une diminution du signal en spectrométrie de masse. Cette étape de rinçage doit être effectuée avec une solution qui ne perturbe pas l'interaction récepteur-analyte. De plus, sur les biopuces Biacore, il n'est possible d'immobiliser qu'un nombre limité de récepteurs dans des microcanaux à l'intérieur de ces biopuces (4 au maximum sur une surface d'environ 1 mm²).

Face à cette situation, l'essor considérable de puces au format « microarrays » permettant le dépôt à leur surface d'un grand nombre de molécules laisse entrevoir une application avantageuse dans le domaine de l'analyse SPR. Ainsi Nedelkov et al. (2006) ont démontré la faisabilité de réaliser un couplage entre la SPR et la MS en « format microarray », en utilisant une biopuce de type Biacore fermée dans un support plastique (CM5) à l'intérieur de laquelle ont été déposés 440 spots. Après analyse SPR dans un appareil Biacore, la puce a été extraite de l'instrument SPR puis lavée et séchée. La biopuce fermée a ensuite été découpée avec un emporte-pièce chaud, conçu pour fondre le plastique autour de la puce afin de ne pas la casser. Ensuite la puce découpée a été déposée à l'aide d'un support dans un spectromètre de masse MALDI-TOF. Il s'agit de la première étude du couplage SPR-MS décrite au format arrays avec la technique SPR Biacore. Il est important de noter qu'avec ce dispositif décrit par Nedelkov, seule l'analyse MS permet une résolution spatiale des différents plots disposés sur la puce. En effet, l'instrument SPR utilisé pour cette expérience ne permet pas de différencier individuellement les spots car le signal SPR est moyenné sur toute la surface de la fluidique (c'est à dire sur 1 mm²). Avec ce système, les plots d'intérêt ne peuvent donc pas être identifiés, ce qui ne permet pas une analyse multiplexe.

Outre le format « microarray », une nouvelle fonctionnalité d'imagerie est apparue permettant d'accéder à une analyse SPR de type multiplexe, c'est-à-dire sur une multiplicité d'échantillons analysés simultanément et en temps réel (Surface plasmon microscopy, B. Rothenhausler, W. Knoll, Nature, 1988, 332, 615-617).

Le couplage des techniques analytiques SPR et MALDI-MS sur une même interface de type biopuce est dépendant de la chimie de surface développée pour fonctionnaliser la biopuce. Cette chimie doit permettre une fixation optimale des récepteurs d'une part, et être compatible avec l'analyse SPR et l'analyse MALDI-MS d'autre part. Dans ce contexte, Nedelkov a proposé l'utilisation de monocouche auto-assemblée (SAM-self assembled monolayer) dès 2002 (Proteomics 2002, 2 441-446) en utilisant des chaînes alcanethiol MUA (acide mercaptoundécanoïque) connues pour former une monocouche organisée sur des surfaces métalliques et particulièrement sur l'or. Dernièrement, un système SPRi-MS au format microarray a été présenté dans lequel la biopuce est fonctionnalisée par les chaînes alcanethiol MUA (Nedelkov, Analytical Chemistry 2007, pp. A-D). Or, ce type de monocouche auto-assemblée de MUA présente l'inconvénient d'être à l'origine d'interactions non-spécifiques dues au caractère hydrophobe des chaînes alkyle.

C'est dans ce cadre que les inventeurs ont élaboré la présente invention, comprenant le couplage en ligne de la SPR avec la spectrométrie de masse, notamment par désorption laser assistée par matrice (ou MALDI-MS). D'autres méthodes de désorption ionisation peuvent être envisagées pour le couplage en ligne SPR-MS se déroulant sur une même surface et au format microarray : la DESI-MS (desorption electrospray ionisation), la FAB-MS (fast atom bombardment) ou la SIMS-MS (secondary ion mass spectrometry). Ces techniques d'analyse MS sont plus particulièrement utilisées pour des analytes de faibles masses (<2000 Da), et n'ont pas à ce jour permis d'effectuer l'analyse de biomolécules de haute masse comme des protéines.

La présente invention a pour objet un procédé permettant de fabriquer une puce qui pourra servir de support commun pour une analyse couplée SPR-MS.

L'invention a également pour objet un procédé d'analyse de biomolécules permettant de réaliser l'ensemble de l'expérience SPR-MS, qui va de l'analyse et de la quantification des interactions par SPR, jusqu'à la caractérisation fine en spectrométrie de masse, sur le même support et au « format microarrays ». L'ensemble de l'expérience SPR-MS peut ainsi être réalisé sans déplacement ni traitement de l'analyte. En effet, l'analyte, dont l'interaction spécifique avec une molécule récepteur immobilisée sur la biopuce est détectée par SPR, est directement analysé par spectrométrie de masse sans étape de microélution. Ainsi l'analyte est préservé de l'action de tampons dénaturants. Etant directement analysé sur la biopuce par spectrométrie de masse, l'analyte ne subit aucune extraction qui engendrerait des pertes. Les tampons utilisés sont compatibles avec les deux analyses SPR et MS.

Les inventeurs ont mis au point un procédé permettant de fonctionnaliser un support solide utilisable en SPR de manière à le rendre compatible avec le couplage de deux techniques d'analyse : la SPR, en particulier SPR par imagerie (ou SPRi) et la spectrométrie de masse (MS), en particulier MALDI-MS.

Le procédé selon l'invention est un procédé de fonctionnalisation de la face métallique d'un support d'analyse (ou puce) destinée aux analyses SPR et MS, la face métallique étant préférentiellement de l'or dans la présente invention. Le procédé comprend le greffage d'une monocouche autoassemblée de poly(oxyde) d'éthylène (POE) directement sur la face métallique dudit support. À cette fin, le POE est directement mis en contact avec la face métallique à fonctionnaliser, cette fonctionnalisation se faisant par greffage chimique.

Avantageusement, le support ainsi modifié possède des qualités permettant l'analyse fine des interactions entre molécules tout en permettant leur analyse structurale. Le support modifié permet notamment de réduire au minimum les interactions non spécifiques de biomolécules vis-à-vis de la monocouche lors de l'étude des interactions des analytes avec les récepteurs immobilisés. La limitation de l'adsorption non-spécifique des analytes est obtenue grâce au choix de la nature de la monocouche autoassemblée composée de motifs oxyde d'éthylène. Il permet également d'améliorer avantageusement le couplage SPR-MS en améliorant le rapport signal sur bruit. Le support modifié autorise aussi avantageusement la digestion protéolytique *in situ* après interaction récepteurs-analytes en SPR. Cette digestion protéolytique donne ainsi accès à une analyse protéomique fine par MS.

Le procédé selon l'invention comprend la fonctionnalisation d'une face métallique d'une puce destinée aux analyses SPR et MS. Comme cela est bien connu de l'homme du métier, les expériences de SPR sont réalisées en fixant des molécules « récepteurs » sur la face métallique fonctionnalisée du support (ou puce) comprenant une face métallique permettant d'enregistrer le phénomène de résonance de plasmons de surface. Ainsi, pour observer un phénomène de résonance de plasmons de surface, il faut utiliser une surface métallique qui peut être en argent, or, platine, aluminium ou cuivre. Avantageusement, la face métallique du support est en or, le métal le plus généralement utilisé en SPR par l'homme de métier.

Les dimensions des supports utilisés en SPR et leur procédé de montage sont connus de l'homme du métier. Ainsi, dans un mode particulier de réalisation, le support est composé d'une lame de verre sur laquelle on dépose la surface métallique, de préférence la surface en or. L'épaisseur de la surface métallique peut notamment être comprise entre 10 nm et 100 nm. Avantageusement, une couche de chrome peut être intercalée entre la lame de verre et la surface métallique. Cette couche de chrome peut avoir une épaisseur comprise entre 1 nm et 5 nm, de préférence entre 1 ou 2 nm.

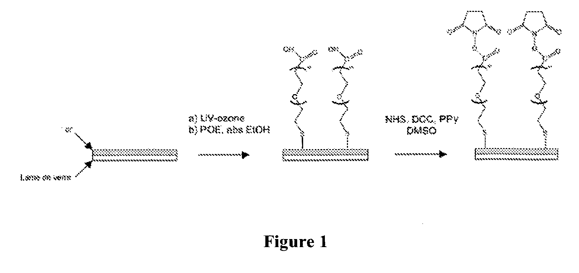

Selon le procédé de la présente invention, la face métallique du support de SPR est fonctionnalisée par greffage chimique du poly(oxyde) d'éthylène (POE) directement sur ladite face métallique. Ce procédé comprend donc un greffage direct du POE, c'est-à-dire qu'une interaction forte est établie entre l'atome de soufre du groupement thiol du POE et la face métallique du support sans molécule intermédiaire. Ces molécules de POE s'organisent par autoassemblage en monocouche. Cette configuration est représentée sur la figure 1.

Dans le cadre de la présente invention, le terme « autoassemblage » ou « autoassemblé » se réfère à la capacité qu'ont les chaînes de POE à s'organiser spontanément les unes par rapport aux autres dans l'espace après le greffage (Surface Science Reports 61 (2006) 445-463*).*

Le terme « monocouche », dans le cadre de la présente invention, couvre des chaînes formant une structure dense et ordonnée dans le plan de la face métallique. Il s'agit typiquement d'une couche monomoléculaire ordonnée rencontrée dans le cas des alcanes thiol.

Le greffage peut être réalisé par tout moyen connu de l'homme du métier. Par exemple, le support comprenant la face métallique peut être plongé directement dans une solution contenant le POE à greffer. Alternativement, la solution de POE peut être déposée uniquement sur la face métallique du support.

La solution contenant le POE comprend le POE à greffer mis en solution dans un solvant adéquat n'entravant pas la réaction d'autoassemblage du POE sur la face métallique du support. Ce solvant peut notamment être de l'éthanol absolu, de l'eau, du dichlorométhane ou du dimethylsulfoxyde. Dans un mode particulièrement préféré de réalisation, le solvant utilisé est de l'éthanol absolu. La concentration en POE dans la solution peut typiquement être comprise entre 0,5 et 10 mM, de préférence entre 1 et 5 mM, et de manière particulièrement préférée la concentration de POE est d'environ 2,5 mM. Cette dernière concentration permet d'atteindre 100 % de greffage.

Le greffage est réalisé à une température comprise de préférence entre 4°C et 100°C, de préférence entre 10°C et 70°C, de manière encore plus préférée entre 15°C et 30°C. Avantageusement, le greffage est réalisé à température ambiante, c'est à dire à 20°C. Les conditions de pression peuvent également être adaptées. Ainsi, la réaction peut être réalisée notamment à une pression comprise entre 10⁻⁵ et 2 atmosphères. De manière préférée, la réaction de greffage est réalisée à pression atmosphérique. Le temps de greffage est d'au moins une heure. De préférence, le temps de greffage est d'au moins 6 heures. Des temps de réaction plus long peuvent être envisagés, notamment au moins 12 heures ou au moins 24 heures. Il est entendu que le support peut être mis en présence du POE à greffer pendant une durée pouvant dépasser plusieurs semaines. Cependant, en pratique, le temps de greffage sera en général inférieur à 15 jours, de préférence inférieur à 7 jours.

Avantageusement, la réaction de greffage est réalisée en plongeant le support dans une solution de POE dissous dans de l'éthanol absolu entre 15°C et 30°C, à une pression de 0,5 à 2 atmosphères pendant au moins 6 heures.

De préférence, la réaction est réalisée de manière à obtenir un taux de recouvrement de POE sur la face métallique du support supérieur à 70 %, de préférence à 80 %, de préférence à 90 %, de préférence à 95 %. De manière encore plus préférée, le taux de recouvrement du support est essentiellement de 100 %. Ce pourcentage correspond au pourcentage d'inhibition de la conductivité de la face métallique après recouvrement avec le POE, par rapport à la conductivité mesurée avant recouvrement.

Avantageusement, le POE utilisé dans le cadre de la présente invention est un composé de formule (I)

A-(CH₂)ₙ-(O-CH₂-CH₂)ₓ-D (I)

dans laquelle :
- n est égal à 1 ou 2;
- x est un nombre entier compris entre 5 à 16;
- A est un groupement permettant l'ancrage du POE par une liaison covalente sur la surface métallique du support ; et
- D est un groupement, éventuellement modifié, permettant la fixation de bio molécules.

Dans un mode particulier de réalisation du procédé de fonctionnalisation, n est égal à 2. Dans un mode préféré de réalisation, x est égal à 8.

Dans un mode de réalisation particulièrement avantageux du procédé de fonctionnalisation selon l'invention, le POE est un POE à courte chaîne, notamment un POE dans lequel n est égal à 2 et x est égal à 8, c'est à dire un composé de formule :

A-(CH₂)₂-(O-CH₂-CH₂)₈-D.

Le groupement A est un groupement permettant l'ancrage de la molécule de POE sur la surface métallique du support. Ainsi, si la surface métallique est en or, argent, platine ou cuivre, A est un groupement thiol (-SH), connu de l'homme du métier pour se greffer sur ces métaux, en particulier sur l'or, le soufre ayant une grande affinité pour l'or. Ainsi, avantageusement, un mode particulier de réalisation de l'invention concerne un procédé tel que défini ci-dessus, dans lequel le métal de la face métallique du support est de l'or, et A est un groupement -SH.

Les molécules « récepteurs » sont, dans le cadre de la présente invention, des molécules d'intérêt biologique, notamment des peptides, sucres ou acides nucléiques. En particulier, les molécules « récepteurs » peuvent être des protéines, oligosaccharides ou oligonucléotides.

Le groupement D permet la fixation de molécules sur le support fonctionnalisé. Le choix du groupement D dépendra donc du type de molécule à immobiliser, ce choix étant largement à la portée de l'homme du métier. On peut citer à titre de groupement D les groupes -COOH, -NH₂, -CHO, entre autres. Le groupement D peut permettre la fixation directe de molécules, ou une fixation indirecte après modification du groupe D.

Dans le cas particulier où les molécules récepteurs sont :
- des protéines, le groupement D ou le groupement D modifié peut être un groupement N-hydroxysuccinimide (NHS), ester-succinimidyl ou ester-sulfonosuccinimidyl qui va former des liaisons amides avec les amines primaires N-terminales des protéines ; un groupement fonctionnalisé maléimide ou iodoacétyl pour réagir avec les protéines thiolées et aboutir à une liaison thioéther ;
- des oligosaccharides, le groupement D ou le groupement D modifié par des méthodes connues par l'homme du métier pour réagir de manière covalente avec l'extrémité réductrice du sucre ;
- des oligonucléotides (ADN ou ARN), le groupement D ou le groupement D modifié peut être un ester-succinimidyl qui va former une liaison amide avec le brin synthétique lui-même fonctionnalisé à l'une de ses extrémités par une fonction amine primaire.

Dans un mode de réalisation particulièrement préféré dans le cadre de l'immobilisation de protéines, le groupement D correspond à un groupe pouvant être modifié en groupe N-hydroxysuccinimide (NHS), notamment le groupe -COOH.

Dans un mode de réalisation particulièrement préféré du procédé de fonctionnalisation selon l'invention, le POE greffé sur la face métallique du support est un POE de formule HS-CH₂-CH₂-(O-CH₂-CH₂)₈-COOH.

Le POE à greffer peut être trouvé dans le commerce, notamment le POE de formule HS-CH₂-CH₂-(O-CH₂-CH₂)₈-COOH, encore appelé O-(2-mercaptoéthyl)-O'-(2-carboxyethy)heptaéthylène glycol. Alternativement, le POE à greffer peut être synthétisé selon des procédés qui sont à la portée de l'homme du métier (J. Am. Chem. Soc. 1993, 115, 10714-10721).

Dans un mode particulier de réalisation, le procédé de fonctionnalisation selon l'invention comprend :
1) le nettoyage préalable du support ;
2) le greffage du POE sur le support nettoyé et autoassemblage en monocouche sur la face métallique du support, tel que décrit ci-dessus ;
3) éventuellement, la modification du groupe D du POE.

Le nettoyage à l'étape 1) ci-dessus est réalisé par tout moyen adapté connu de l'homme du métier. En particulier, ce nettoyage peut notamment être réalisé par traitement UV-ozone de la surface métallique à fonctionnaliser. Alternativement, le support peut être nettoyé avec de l'acide sulfochromique.

Après l'étape 2) de greffage du POE sur le support, le support fonctionnalisé peut être conservé de préférence à sec et/ou de préférence à froid (environ 4°C), tel qu'au réfrigérateur (notamment en atmosphère confinée), avant traitement ultérieur ou avant son utilisation pour l'analyse par SPR et/ou MS.

L'étape 3), qui est optionnelle, correspond à la modification du groupe D du POE. La nécessité d'une modification dépendra du type de groupement D à l'extrémité du POE greffé sur la face métallique, comme cela est bien connu de l'homme du métier. Par exemple, si D représente un groupe -COOH, ce dernier peut être modifié en groupe N-Hydroxysuccinimide (NHS), dans les conditions standards connues pour ce type de réaction. Le groupe NHS employé pour la modification de l'extrémité du POE est bien connu pour sa réactivité en tant qu'agent de couplage des protéines (Macromolecules 35 (2002) 581-584*).*

Après l'étape 3 de modification optionnelle du groupe D du POE, le support fonctionnalisé peut être conservé (de préférence à sec), notamment au réfrigérateur (en particulier en atmosphère confinée), avant utilisation dans une analyse par SPR, éventuellement suivie d'une analyse par MS.

L'invention concerne en outre l'utilisation d'un POE de formule (I) telle que définie ci-dessus, selon toutes les variantes envisagées, pour fonctionnaliser la face métallique d'un support d'analyse par SPR.

L'invention concerne également un support fonctionnalisé d'analyse par SPR comprenant une face métallique sur laquelle est greffé un POE tel que défini ci-dessus. A ce titre, le support fonctionnalisé selon l'invention est notamment un support fonctionnalisé obtenu selon le procédé décrit ci-dessus. Il peut comprendre en outre les molécules « récepteurs » immobilisées sur le support fonctionnalisé via une liaison par l'intermédiaire de la monocouche autoassemblée de poly(oxy)éthylène, en particulier du groupe D ou du groupe D modifié de POE, notamment fonctionnalisé par un groupement NHS. Ainsi, le support comprend des molécules « récepteurs » sous la forme d'un réseau de plots. Ce réseau de plots présente l'avantage de pouvoir permettre l'analyse spécifique des interactions d'une molécule « récepteur » donnée avec un échantillon d'analytes, notamment dans le cadre d'une expérience de SPR ou de MS.

L'invention concerne en outre l'utilisation d'un support fonctionnalisé tel que décrit ci-dessus, dans une analyse par SPR, notamment dans une analyse par SPRi. Dans ce cadre, des molécules « récepteurs » sont immobilisées sur le support fonctionnalisé via une liaison par l'intermédiaire du groupe D ou du groupe D modifié, notamment fonctionnalisé par un groupement NHS.

Le protocole d'immobilisation des molécules « récepteurs » dépend du type de groupement D du POE greffé sur le support et du type de molécules « récepteurs » à immobiliser, comme cela est bien connu de l'homme du métier.

Parmi les molécules récepteurs, on peut notamment citer les protéines (antigènes, anticorps, peptides), acides nucléiques (oligonucléotides ADN, ARN) et glucides (oligosaccharides). Ces différents types de molécules peuvent être immobilisés sur le support en choisissant de manière adéquate le groupement D du POE greffé sur le support.

Dans le mode particulier de réalisation où le groupe D est modifié en un groupement NHS ou un dérivé du NHS, il est nécessaire de procéder à une étape d'inactivation des groupements NHS n'ayant pas réagi à l'issue de l'étape d'immobilisation des molécules « récepteurs ». Une solution de lysine peut notamment être employée à cette fin. La lysine est présente dans cette solution à une concentration comprise entre 10 µM et 1mM.

Avantageusement, les molécules « récepteurs » sont immobilisées sur le support fonctionnalisé décrit ci-dessus afin de procéder à une analyse SPRi. Comparé à l'analyse SPR classique, l'analyse SPRi donne accès au format multiplexe, c'est-à-dire l'analyse simultanée (détection, quantification, visualisation) en temps réel et sans marquage d'un grand nombre d'échantillons. Pour ce faire, les molécules « récepteurs » sont immobilisées à la surface du support fonctionnalisé sous la forme d'un réseau de plots dont la composition en molécules immobilisées est connue et ordonnée. Ce réseau donne accès à la résolution spatiale en SPR, c'est-à-dire que les plots d'intérêt sont visualisés en SPR grâce à l'imagerie.

Cette résolution spatiale sera conservée lors de l'analyse MALDI MS. Chaque plot pourra être interrogé individuellement par l'analyseur MS, ainsi chaque analyte retenu spécifiquement sur le support pourra être identifié en MS.

Pour réaliser l'analyse par SPR, notamment par SPRi, le support fonctionnalisé sur lequel des molécules récepteurs ont été immobilisées est placé dans un analyseur SPRi, ou SPR classique. Les données SPR, ou SPRi, sont ensuite acquises et exploitées.

L'avantage majeur du support fonctionnalisé selon la présente invention est qu'il permet un couplage simplifié et amélioré entre une analyse SPR et une analyse MS d'une part, et qu'il minimise les interférences dues à des liaisons non-spécifiques sur la surface d'autre part, notamment observées lors de l'utilisation d'autres supports fonctionnalisés par le MUA décrit précédemment. En effet, après analyse SPR, et avantageusement SPRi, le support fonctionnalisé sur lequel sont immobilisées les molécules « récepteurs » est retiré de l'analyseur SPR, ou SPRi, et est placé directement (sans étapes intermédiaires) dans un spectromètre de masse MALDI MS, sans destruction du support fonctionnalisé, et sans microélution ni changement de solution tampon des analytes retenus spécifiquement au cours de l'analyse SPR. Ceci est particulièrement avantageux puisque les solvants utilisés classiquement pour éluer les molécules fixées sur le support SPR ne sont pas compatibles avec la MS et nécessite donc une étape supplémentaire d'échange de tampon. Cette étape supplémentaire prend du temps et peut entraîner des contaminations et une perte de matériel.

Ainsi, le support fonctionnalisé selon l'invention permet de mettre en place un couplage SPR-MS simplifié, et avec des rendements améliorés. L'invention concerne donc également l'utilisation d'un support modifié tel que décrit ci-dessus pour réaliser l'analyse des interactions moléculaires d'un échantillon disposé sur le support par résonance des plasmons de surface couplée à l'analyse structurale dudit échantillon disposé sur le même support par spectrométrie de masse, de préférence par spectrométrie de masse MALDI.

En d'autres termes, l'invention concerne aussi l'utilisation d'un support fonctionnalisé tel que décrit ci-dessus, pour réaliser deux analyses consécutives : une analyse par SPR puis une analyse par spectrométrie de masse.

L'invention concerne également l'utilisation d'un support fonctionnalisé tel que décrit ci-dessus dans une expérience de spectrométrie de masse, notamment de type MALDI, notamment de type MALDI-TOF.

Dans le cas d'une analyse par spectrométrie MALDI, l'homme du métier sait que les échantillons présents sur le support doivent être enrobés dans une matrice adéquate (Hillenkamp, F.; Karas, M.; Beavis, R. C.; Chait, B. T. Analytical Chemistry 1991, 63, A1193-A1202).

L'homme du métier pourra mettre en oeuvre le support selon l'invention dans une analyse protéique des analytes retenus sur les molécules « récepteurs » immobilisées sur le support fonctionnalisé, notamment en réalisant une étape de digestion enzymatique localisée *in situ* directement sur la surface, ce qui permettra d'obtenir soit l'empreinte peptidique d'une protéine par MALDI MS, ou sa séquence par MALDI-MS/MS.

L'invention concerne plus particulièrement un procédé de couplage d'une analyse par SPR, notamment SPRi, avec une analyse par MS, notamment MALDI, comprenant :
1) l'immobilisation d'une ou plusieurs molécules « récepteurs » sur un support fonctionnalisé selon l'invention ; puis
2) la mise en place du support dans un analyseur SPR et l'analyse par SPR des interactions entre la ou les molécules « récepteurs » immobilisées et un échantillon d'analytes ; puis
3) le retrait du support de l'analyseur SPR et sa mise en place dans un spectromètre de masse, et l'analyse structurale par MS des analytes retenus spécifiquement par les molécules « récepteurs » au cours de l'analyse SPR.

La MS va permettre d'identifier structuralement les analytes qui ont été retenus spécifiquement par les molécules récepteurs, elles-mêmes liées de façon covalente au support fonctionnalisé selon l'invention.

Par « analyte » on désigne, dans le cadre de la présente invention, des substances ou composés chimiques soumis à une procédure analytique pour en détecter la présence et/ou en mesurer une caractéristique (ex : teneur, concentration). Il s'agit notamment de substances ou composés chimiques dont on veut déterminer la capacité à interagir avec les molécules « récepteurs » immobilisées sur un support tel que décrit ci-dessus. Les analytes peuvent correspondre à un mélange dont le contenu n'est pas connu, par exemple un extrait protéique, glucidique, d'acides nucléiques, etc. Le mélange pourrait également contenir un mélange de protéines, glucides, acides nucléiques, etc. connus.

Au cours de l'étape 1) d'immobilisation, une ou plusieurs molécules « récepteurs » peuvent être immobilisées. A ce titre, plusieurs dizaines, plusieurs centaines, voire plusieurs milliers de molécules « récepteurs » différentes peuvent être immobilisées. Avantageusement, les molécules « récepteurs » sont immobilisées de manière discrète et ordonnée sur le support de l'invention sous forme de plots. Ainsi, la position de chacune de ces molécules « récepteurs » est directement adressable, rendant possibles les analyses SPR et MS de manière individuelle, plot par plot (c'est à dire, molécule « récepteur » par molécule « récepteur »). Ainsi, le support fonctionnalisé permet avantageusement le multiplexage au cours de l'analyse MS qui suit l'analyse SPR.

Dans un mode de réalisation de l'invention particulièrement préféré, les analyses en MS sont réalisées par MALDI-MS. Dans ce cadre, une matrice compatible avec l'analyse MALDI est déposée sur le support avant son introduction dans le spectromètre de masse.

Les inventeurs du présent procédé ont aussi pu démontrer que le support fonctionnalisé selon l'invention permet de réaliser une digestion enzymatique localisée sur chacun des plots correspondant à une molécule « récepteur » immobilisée. Ainsi l'enzyme choisie peut être déposée directement sur les plots. Doté de cette fonctionnalité supplémentaire, le procédé de couplage en ligne SPRi-MS décrit dans la présente invention offre à l'utilisateur le choix de deux types d'analyse MS sur le support fonctionnalisé : l'une sans digestion enzymatique conduisant à la détermination de la masse entière des analytes ayant été retenus spécifiquement sur les récepteurs, et l'autre incluant un traitement enzymatique *in situ* des analytes retenus (protéines, glucides, acide nucléiques), et conduisant à des données structurales plus détaillées.

Ainsi, l'invention concerne également un procédé de couplage d'une analyse par SPR, notamment SPRi, avec une analyse par MS, notamment MALDI-MS, ou MS/MS comprenant :
1) l'immobilisation d'une ou plusieurs molécules « récepteurs » sur un support fonctionnalisé selon l'invention ; puis
2) la mise en place du support dans un analyseur SPR et l'analyse par SPR des interactions entre la ou les molécules « récepteurs » immobilisées et un échantillon d'analytes ;
3) la digestion enzymatique localisée *in situ* des analytes retenus sur les plots des molécules « récepteurs » immobilisées sur le support fonctionnalisé, puis la mise en place du support dans un spectromètre de masse, et l'analyse structurale par MS ou MS/MS des produits de digestion de la ou des analytes présents sur le support fonctionnalisé.

Par exemple, dans le cas d'une analyse protéique, la protéolyse directement sur la surface permettra d'obtenir soit l'empreinte peptidique d'une protéine par MALDI MS ou sa séquence par MALDI-MS/MS. Doté de cette fonctionnalité, le procédé de couplage en ligne SPRi-MS décrit dans la présente invention donne ainsi l'accès à l'analyse structurale fine, notamment dans le cas d'analyses protéomiques.

En outre ces deux analyses peuvent être combinées successivement, sur le même support fonctionnalisé selon la séquence suivante : analyse SPR et capture de l'analyte, caractérisation de la masse entière de l'analyte par MS, suivi d'une digestion enzymatique de l'analyte et d'une seconde analyse par MS. En effet, suite au couplage en ligne SPRi MALDI-MS donnant accès à la masse entière de l'analyte, les auteurs ont pu démontrer qu'il était possible d'éliminer la matrice nécessaire à la caractérisation en MALDI-MS, afin de procéder à une digestion enzymatique des molécules liées au support *in situ.* L'élimination de la matrice est effectuée par rinçage avec la solution de solubilisation de la matrice, qui pour cet usage est dépourvue d'acide. On peut notamment utiliser une solution de solubilisation du type acétonitrile ou éthanol.

Dans ce cas, l'invention concerne un procédé de couplage d'une analyse par SPR, notamment SPRi, avec une analyse par MS, notamment MALDI-MS, comprenant :
1) l'immobilisation d'une ou plusieurs molécules « récepteurs » sur un support fonctionnalisé selon l'invention ; puis
2) la mise en place du support dans un analyseur SPR et l'analyse par SPR des interactions entre la ou les molécules « récepteurs » immobilisées et un échantillon d'analytes ; puis
3) le retrait du support de l'analyseur SPR et sa mise en place dans un spectromètre de masse, et l'analyse structurale par MS des analytes retenus spécifiquement par les molécules « récepteurs » au cours de l'analyse SPR ; puis
4) la digestion enzymatique localisée *in situ* des analytes retenus sur les plots des molécules « récepteurs » immobilisées sur le support fonctionnalisé, l'enrobage des échantillons dans une matrice adéquate pour une analyse par MALDI-MS, la mise en place du support dans un spectromètre de masse, et l'analyse structurale par MALDI-MS ou MALDI-MS/MS des produits de digestion de la ou des molécules présentes sur le support fonctionnalisé.

Dans un mode particulier de réalisation, l'analyse structurale de l'étape 3) ci-dessus est réalisée par MALDI-MS. Dans ce cas, après cette analyse, la matrice utilisée pour la MALDI-MS est éliminée avant l'étape 4.

### DESCRIPTION DES FIGURES

La figure 1 est un schéma représentant un support selon l'invention, comprenant une face métallique en or et les étapes de fonctionnalisation de cette surface. La surface est fonctionnalisée avec un POE de formule HS-CH₂-CH₂-(O-CH₂-CH₂)₈-COOH, puis ce POE est modifié avec un groupement NHS.
La figure 2 représente les résultats de la comparaison de monocouches autoassemblées POE-NHS à des monocouches autoassemblées MUA-CDI greffées à la surface d'or de biopuces SPR-MS.
La figure 3 représente une expérience SPRi sur une biopuce ayant subit un traitement de surface POE-NHS selon l'invention et montrant la spécificité d'interaction des analytes pour leurs récepteurs.
La figure 4 représente un exemple de couplage SPRi-MALDI-TOF MS sur une biopuce de type monocouche POE-NHS autoassemblée sur une surface d'or.
La figure 5 représente un exemple d'analyse protéomique après une expérience SPRi puis analyse par MALDI-TOF MS des analytes digérés *in situ.* L'expérience est réalisée sur une biopuce de type monocouche autoassemblée POE-NHS sur une surface d'or.
La figure 6 représente un exemple d'analyse protéomique après une expérience SPRi puis séquençage par MALDI-MS/MS des peptides issus de la digestion *in situ* de la protéine ovalbumine capturée sur la biopuce. L'expérience est réalisée sur une biopuce de type monocouche autoassemblée POE-NHS sur une surface d'or. Les spectres MALDI-MS/MS de peptides issus de la digestion sur biopuce et l'identification de la protéine retenue à l'aide de l'outil informatique « Mascot » sont représentés sur la figure.

### EXEMPLES

### Exemple 1 : Protocole de préparation de lamelle monocouche autoassemblée POE-NHS

### Dimension de la biopuce amovible

Lame de verre de 500 µm d'épaisseur, de dimension 12 mm x 28 mm.
Epaisseur de chrome : 1 à 2 nm.
Epaisseur de la couche d'or déposée à la surface : 50 nm.

### Caractéristiques du POE

Le POE bifonctionnalisé utilisé est d'origine commerciale (Sigma-Aldrich ; ref: 672688). Il s'agit du O-(2-Mercaptoethyl)-O'-(2-carboxyethy)heptaéthylène glycol, composé de 8 motifs OE [(oxy)éthylène] et fonctionnalisé à ses deux extrémités :

HS-CH₂-CH₂-(O-CH₂-CH₂-)₈-COOH

(M=458,56 g.mol-1)

La fonction thiol (mercapto) permet l'ancrage du polymère sur la face métallique de la lame de verre. La fonction acide carboxylique à l'autre extrémité autorise la fonctionnalisation de la chaîne, nécessaire à la liaison covalente des biomolécules « récepteurs ».

### Protocole de préparation du dépôt de POE-NHS sous forme de monocouche autoassemblée à la surface de la biopuce

Après une étape préalable de nettoyage, le POE est d'abord déposé pour former une monocouche auto-assemblée, puis dans l'étape suivante son extrémité carboxyle est fonctionnalisée par le groupe N-hydroxysuccinimide (cf. schéma figure 1).

### 1) Nettoyage des lamelles par traitement UV-ozone

Des lamelles de verre recouvertes d'or dont les dimensions sont données ci-dessus sont déposées sur du papier optique, face dorée sur le dessus et un traitement UV-ozone est réalisé pendant 1h.

### 2) Dépôt du POE : protocole pour une lamelle

Dans un bécher de 100 mL, 3 mg (6,54.10⁻⁶ mol) de HS-CH₂-CH₂-(O-CH₂-CH₂-)₈-COOH sont dissous dans 2,6 mL d'éthanol absolu (concentration finale du POE 2,5mM ou 1,15mg/mL). La solution est homogénéisée dans le bécher, puis une lamelle est immergée dans celui-ci, directement après le traitement UV-ozone.

Le bécher est ensuite couvert par 3 couches de parafilm pour éviter l'évaporation. Le bécher est ensuite mis sous agitation pendant 6 heures (vitesse Rocking Platform=20). Après cette étape, la lamelle traitée avec le POE est récupérée et l'excédent de solution est absorbé sur du papier buvard. La lamelle traitée est ensuite rincée dans deux bains d'éthanol absolu, séchée puis stockée au réfrigérateur en attendant de réaliser la réaction de fonctionnalisation avec le N-hydroxysuccinimide (NHS).

### 3) Fonctionnalisation du POE par le N-hydroxysuccinimide

Une solution de 1,09 mL de DMSO contenant 27,6 mg (2,4.10⁻⁴ mol) de N-hydroxysuccinimide, 53 mg (2,6.10⁴ mol) de N,N'-dicyclohexylcarbodiimide et 3,5 mg (0,23.10⁻⁴ mol) de 4-pyrrolidinopyridine est préparée dans un bécher de 100 mL. La solution est ensuite homogénéisée.

La lamelle traitée précédemment avec le POE est alors déposée dans la solution de DMSO fraîchement préparée, et le bécher est couvert par deux couches de parafilm. La réaction est ensuite réalisée en laissant la solution agir pendant 24 heures sous agitation (vitesse Rocking Platform = 20). L'excédent de solution est enlevé sur du papier buvard puis la lamelle est rincée une fois dans un bain de DMSO puis cinq fois dans des bains d'eau ultra pure et une fois dans un bain d'éthanol absolu. La lamelle ainsi traitée est alors séchée puis stockée au réfrigérateur à sec avant utilisation.

### Exemple 2 : Présentation d'une expérience SPRi sur une biopuce de type monocouche autoassemblée POE-NHS à la surface d'or.

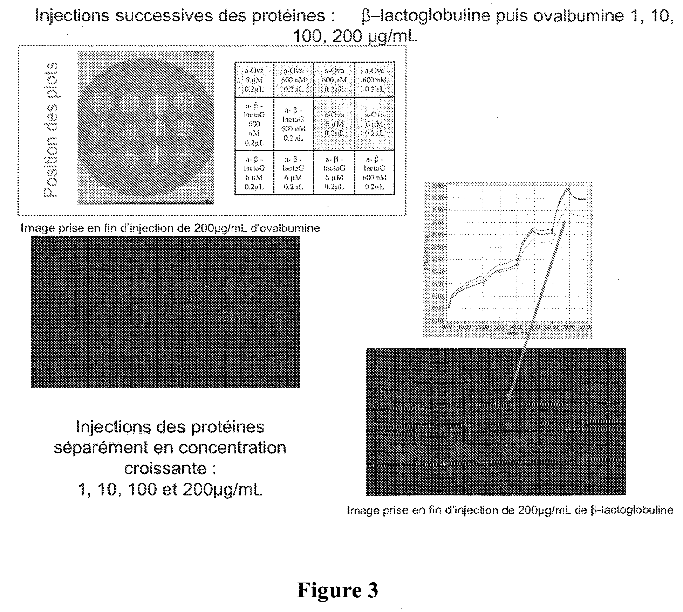

Cet exemple traite d'une expérience SPRi sur une biopuce ayant subit un traitement de surface POE NHS selon l'invention et montrant la spécificité d'interaction des analytes pour leurs récepteurs. (cf figure 3)

Cette expérience porte sur la capacité d'adsorption de deux analytes protéiques, l'ovalbumine et la β-lactoglobuline, sur des plots d'anticorps anti-ovalbumine et anti-β-lactoglobuline (600nM et 6µM) immobilisés sur la surface fonctionnalisée, par comparaison avec des mesures faites sur des zones sans anticorps (zones correspondant à la chimie POE-NHS sans anticorps) et où aucune adsorption d'analytes n'est enregistrée.

Les conditions de l'expérience sont les suivantes :
L'interaction de l'ovalbumine et de la β-lactoglobuline avec les anticorps anti-ovalbumine et anti-β-lactoglobuline immobilisés sur la surface est suivie par SPRi. En début d'expérience, il est procédé à l'injection de lysine (100µM) afin de neutraliser les groupements NHS de la surface fonctionnalisée POE qui n'ont pas réagi avec les anticorps. Ensuite, les deux protéines ovalbumine et β-lactoglobuline (50 µg/mL) sont injectées successivement au débit de 50 µL/min. Des solutions de concentration croissante de β-lactoglobuline (1, 10, 100 et 200µg/mL) puis d'ovalbumine de concentration croissante (1, 10, 100 et 200µg/mL) sont injectées. Le tampon de course est de l'acétate ammonium 10mM pH 7.5. Les images montrées illustrent l'adsorption des protéines sur leur anticorps respectif pour des concentrations de 200µg/mL. Aucune protéine n'est adsorbée sur la surface fonctionnalisée POE-NHS inactivée par la lysine.

### Exemple 3 : Présentation d'un couplage SPRi- MALDI-TOF MS sur une biopuce de type monocouche autoassemblée POE-NHS sur la surface d'or.

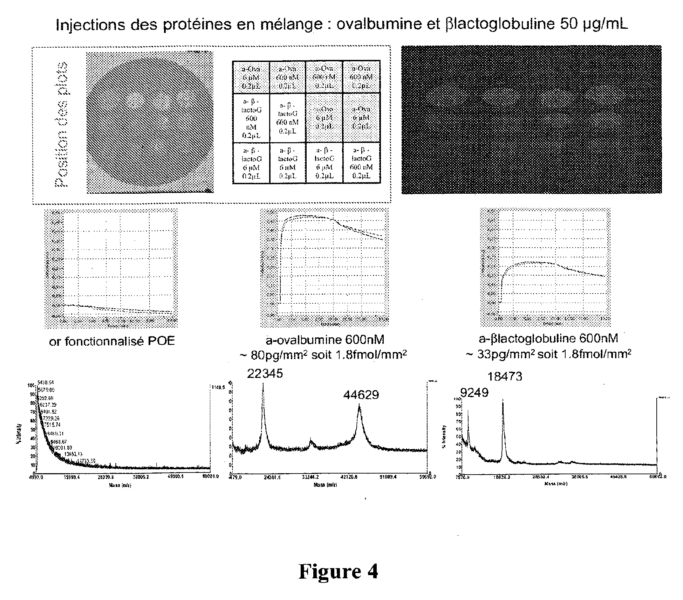

Cet exemple traite du couplage SPRi-MALDI-TOF MS sur une biopuce ayant subit un traitement de surface selon l'invention. (cf figure 4)

Cet exemple porte sur l'analyse SPR de l'interaction de l'ovalbumine et de la β-lactoglobuline avec leur anticorps respectif anti-ovalbumine et anti-β-lactoglobuline (600 nM et 6 µM) immobilisés sur la surface fonctionnalisée sous forme de plots, et sur la détection des analytes protéiques par MALDI-TOF MS.

Les conditions de l'expérience sont les suivantes. L'interaction de l'ovalbumine et de la β-lactoglobuline avec les anticorps anti-ovalbumine et anti-β-lactoglobuline immobilisés sur la surface est suivie par SPRi. En début d'expérience, il est procédé à l'injection de lysine (100µM) afin de neutraliser les groupements NHS de la surface fonctionnalisée POE qui n'ont pas réagit avec les anticorps. Puis, un mélange des deux protéines ovalbumine et β-lactoglobuline (50 µg/mL) est injecté au débit de 50 µL/min. Le début de l'injection du mélange des deux protéines correspond au temps t=0. Ensuite, le support fonctionnalisé est rincé pendant 10 min (t=9 min à t=19 min) par le tampon de course acétate ammonium 10mM pH 7.5. Après avoir procédé à l'enregistrement des données d'interaction par SPRi, la biopuce est extraite de l'instrument SPR puis séchée.

L'image montrée sur la figure 4 illustre l'adsorption des protéines sur leurs anticorps respectifs après 9 minutes d'injection. Les spectres de masse MALDI-TOF sont réalisés en mode linéaire positif (100 tirs répétés et 500 accumulations ; tension d'accélération 25kV ; tension appliquée à la grille 93% ; retard à l'extraction 450ns ; intensité du laser 2800). La matrice MALDI choisie est HABA [acide 2(4-hydroxyphenylazo)benzoïque], 10⁻¹M dans 50/50 eau/acétonitrile 0.1% TFA. La matrice est déposée sur les plots d'anticorps ainsi que sur une zone d'or fonctionnalisée POE-NHS-Lys mais sans anticorps.

Aucune interaction n'est détectée sur le support fonctionnalisé POE-NHS et neutralisé par la lysine. Les anticorps anti-ovalbumine et anti-β-lactoglobuline présentés dans l'exemple ont retenu spécifiquement 1.8 fmol/mm² de protéine. Les spectres de masse MALDI-TOF obtenus dans cet exemple présentent un pic correspondant à un ion monochargé attribué d'après sa masse à la protéine retenue spécifiquement sur son anticorps.

### Exemple 4 : Comparaison de types de monocouches autoassemblées POE-NHS vs MUA-CDI greffées à la surface d'or de biopuces SPR-MS

Cet exemple traite de la comparaison de la chimie de surface de type monocouche autoassemblée POE selon l'invention à la chimie de surface décrite le plus couramment dans la littérature en SPR, c'est-à-dire une fonctionnalisation par MUA

Cette comparaison porte sur la capacité d'adsorption de la β-lactoglobuline sur des plots d'anticorps anti-β-lactoglobuline disposés sur les deux types de surface fonctionnalisée, ainsi que sur le niveau de l'adsorption non-spécifique mesurée en SPRi sur des zones sans anticorps.

Les conditions de l'expérience sont les suivantes. L'interaction de la β-lactoglobuline avec l'anticorps anti-β-lactoglobuline (6µM) immobilisé sur les deux types de surface est suivie par SPR. En début d'expérience, il est procédé à l'injection de lysine (100µM) afin de neutraliser les groupements NHS et CDI (carbonyldiimidazole) des surfaces fonctionnalisées POE et MUA respectivement, qui n'ont pas réagi avec les anticorps. Ensuite une solution de β-lactoglobuline (50 µg/mL) a été injectée au débit de 50 µl/min. Le début de l'injection de la β-lactoglobuline correspond au temps t=0. Les supports fonctionnalisés sont ensuite rincés pendant 5 min (t=9min à t=14 min) par le tampon de course acétate ammonium 10mM pH 7.5.

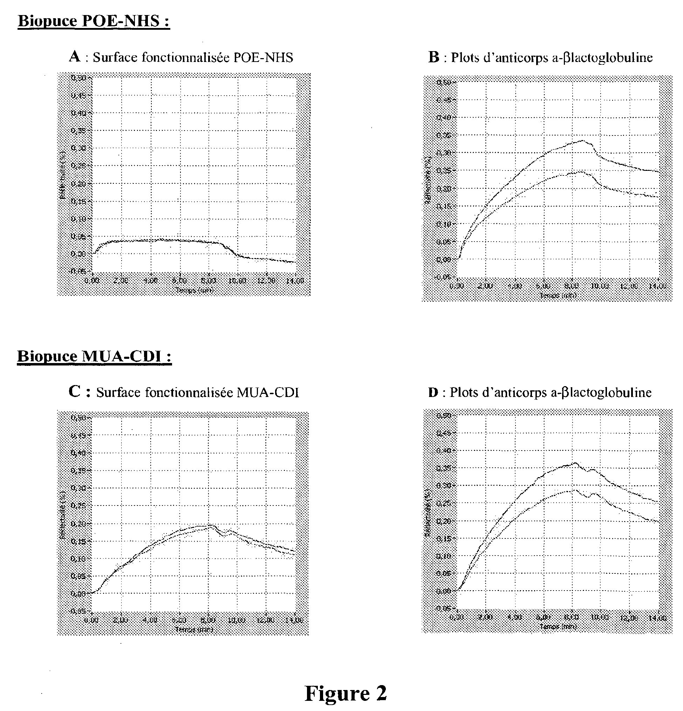

Les résultats sont représentés sur la figure 2.

On constate que la zone fonctionnalisée par MUA-CDI (fig. 2C, sans anticorps) retient des protéines de manière non spécifique et ceci malgré un rinçage de 5 minutes. Cette adsorption non spécifique doit donc être prise en compte lors de la quantification de protéines adsorbées spécifiquement sur l'anticorps (fig. 2D). En outre, cette adsorption non spécifique peut être une source de difficulté lors de l'identification par spectrométrie de masse des protéines retenues spécifiquement sur les sondes.

Dans le cas de la chimie à base de POE proposée dans la présente invention, cette adsorption de protéines sur la zone non traitée par l'anticorps est totalement négligeable (fig. 2A) et ne nécessite aucun ajustement lors de la quantification de protéines adsorbées spécifiquement sur l'anticorps (fig. 2B).

Une analyse MS après une expérience SPRi selon le protocole décrit ci-dessus sur les surfaces d'or fonctionnalisées POE-NHS et MUA-CDI conduit à la détection de protéine sur les plots d'anticorps immobilisé selon les deux traitements de surface. En revanche, la surface fonctionnalisée MUA-CDI dépourvue de plots d'anticorps qui a été inactivée par un traitement à la lysine conduit à la détection de protéine en MS MALDI. Ces résultats lors de l'analyse en SPRi suivie par une analyse MALDI illustrent la capacité de la fonctionnalisation MUA-CDI à engendrer des liaisons aspécifiques qui nuisent aux analyses.

### Exemple 5 : Digestion protéolytique in situ lors d'une analyse SPR-MALDI-MS

### Présentation d'une analyse protéomique après une expérience SPRi et analyse par MALDI-TOF MS des analytes digérés in situ, expérience réalisée sur une biopuce de type monocouche auto-assemblée POE-NHS à la surface d'or.

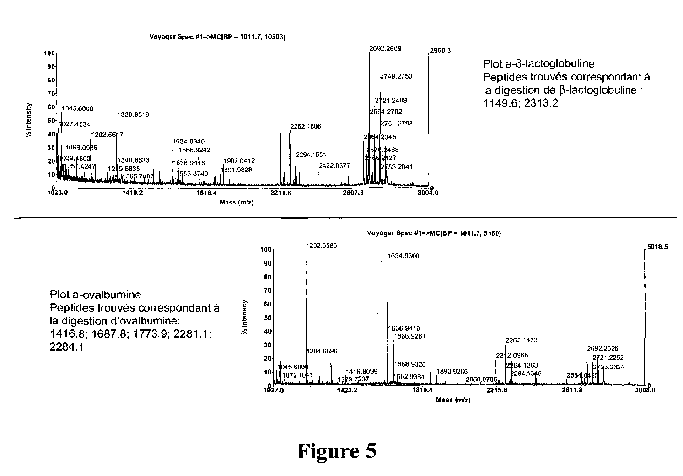

Cet exemple traite d'une digestion tryptique, analysée en ligne par MALDI-TOF MS à la suite de la capture de l'analyte par l'anticorps immobilisé lors de l'analyse SPRi sur le support fonctionnalisé selon l'invention. (cf figure 5)

Cette expérience porte sur la capacité d'accéder à des données protéomiques suite à un couplage en ligne SPRi - traitement tryptique - MALDI-MS. Cette expérience concerne l'interaction entre l'ovalbumine et la β-lactoglobuline sur des plots d'anticorps anti-ovalbumine et anti β-lactoglobuline déposés ou immobilisés sur la surface fonctionnalisée.

Les conditions de l'expérience sont les mêmes que dans l'exemple 2. L'interaction de l'ovalbumine et de la β-lactoglobuline avec les anticorps anti-ovalbumine et anti-β-lactoglobuline (6 µM) immobilisés sur la surface est suivie par SPRi. Pour cela, (comme dans exemple 2) en début d'expérience, il est procédé à l'injection de lysine (100µM) afin de neutraliser les groupements NHS de la surface fonctionnalisée POE qui n'ont pas réagi avec les anticorps. Puis 500µL d'un mélange des deux protéines ovalbumine et β-lactoglobuline (50 µg/mL) est injecté au débit de 50 µL/min. Le début de l'injection du mélange des protéines correspond au temps t=0. Ensuite, le support fonctionnalisé est rincé pendant 10 min (t=8 min à t=18 min) par le tampon de course acétate ammonium 10mM pH 7.5. À l'issue de l'enregistrement des données SPRi, la biopuce est extraite de l'instrument SPR puis séchée. Les quantités retenues sont de 40 pg/mm² de β lactoglobuline sur les plots anti-β-lactoglobuline et de 48 pg/mm² d'ovalbumine sur les plots anti-ovalbumine. La protéolyse est réalisée *in situ* par addition de trypsine déposée sur les plots d'anticorps et incubation de la puce 1h dans une enceinte humide à 37°C. Les spectres de masse MALDI-TOF sont réalisés en mode reflectron (100 tirs répétés et 500 accumulations ; tension d'accélération 20kV ; tension appliquée à la grille 68% ; retard à l'extraction 350ns ; intensité du laser 2400). La matrice MALDI choisie est HCCA [acide α-cyano-4-hydroxycinnamique], 10⁻¹M dans 50/50 eau/acétonitrile 0.1% TFA. La matrice est déposée sur les plots d'anticorps. Les spectres de masse obtenus montrent différents signaux parmi lesquels peuvent être identifiés ceux correspondants aux produits de digestion des analytes.

### Exemple 6 : Identification d'une protéine capturée sur la biopuce SPRi par digestion protéolytique in situ et séquençage par spectrométrie de masse en tandem MALDI-MS/MS

Cet exemple est une présentation d'une analyse protéomique par MALDI-MS et MALDI-MS/MS réalisée après une expérience SPRi. Le séquençage de peptides formés par la protéolyse *in situ* d'une protéine spécifiquement retenue permet d'en déterminer sans ambiguïté l'identité, l'expérience étant réalisée sur une biopuce de type monocouche auto-assemblée POE-NHS à la surface d'or.

Cet exemple traite donc du séquençage en ligne de peptides par MALDI-MS/MS à la suite de la capture d'une protéine par l'anticorps immobilisé lors de l'analyse SPRi et d'une digestion tryptique *in situ* sur le support fonctionnalisé selon l'invention.

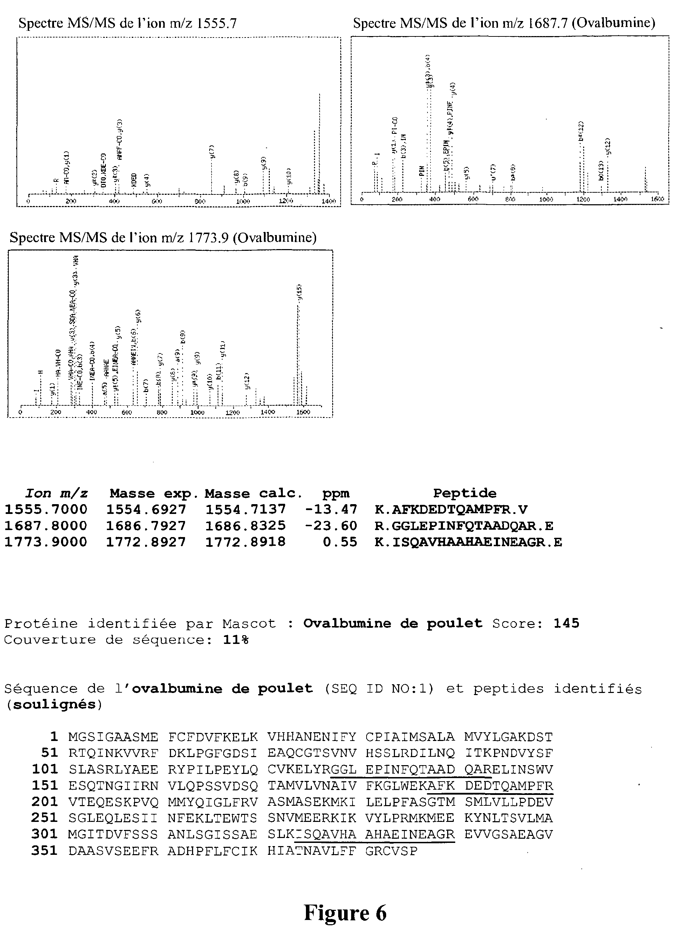

Cette expérience illustre la possibilité d'identifier une protéine spécifiquement retenue suite à un couplage en ligne SPRi - traitement trypsique - MALDI-MS et MALDI-MS/MS. L'exemple servant d'illustration de principe concerne l'interaction entre l'ovalbumine et l'anticorps anti-ovalbumine immobilisé sur la surface fonctionnalisée (*cf.* figure 6).

L'immobilisation des anticorps anti-ovalbumine sur la surface (préparée selon l'exemple 1) puis l'interaction de l'ovalbumine avec les anticorps anti-ovalbumine est suivie par SPRi. L'ovalbumine (200µL, 100 µg/mL) est injectée au débit de 50 µL/min. À l'issue de l'enregistrement des données SPRi, la biopuce est extraite de l'instrument SPR puis séchée. La quantité d'ovalbumine retenue est de ∼29 pg/mm² sur les plots d'anticorps anti-ovalbumine. La protéolyse est réalisée *in situ* par dépôt de trypsine (0.5µg/plot) et incubation de la puce 1h à température ambiante. Le milieu réactionnel de protéolyse est maintenu en ajoutant sur les plots 2 fois 1µL de tampon acétate d'ammonium 0.1 M, pH 8 (tampon de dilution de la trypsine) toutes les 20 minutes. Des spectres de masse MALDI-MS et MALDI-MS/MS sont ensuite enregistrés à partir de la surface de la même biopuce (intensité du laser 4900 ; tension d'accélération Source 1 : 8kV ; Cellule de collision 7kV; Source 2 : 15kV). La matrice MALDI choisie est HCCA [acide α-cyano-4-hydroxycinnamique], 10⁻¹M dans 50/50 eau/acétonitrile 0.1% TFA. Les spectres de masse MALDI-MS obtenus montrent différents signaux parmi lesquels 9 peptides correspondent aux produits de digestion de l'ovalbumine (m/z 1345.73 ; 1555.72 ; 1571.71 ; 1581.72 ; 1597.71 ; 1687.83 ; 1773.89 ; 1858.96 ; 2008.94). Parmi ces ions, les trois plus abondants ont été sélectionnés pour des expériences de MS/MS (m/z 1555.7 ; 1687.8 et 1773.9). Les ions fragments obtenus à partir de chacun de ces peptides ont permis d'identifier à partir du logiciel MASCOT bien connu de l'homme du métier (http://www.matrixscience.com) les séquences peptidiques suivantes
m/z 1555.7 : AFKDEDTQAMPFR (SEQ ID NO:2);
m/z 1687.8 : GGLEPINFQTAADQAR (SEQ ID NO:3); et
m/z 1773.9 : ISQAVHAAHAEINEAGR (SEQ ID NO:4).

Une recherche en banque de données (SwissProt 56.2) à l'aide du logiciel MASCOT permet d'identifier précisément à partir de ces trois séquences peptidiques, la protéine spécifiquement retenue sur les plots (*cf.* figure 6). L'ovalbumine est identifiée sans ambiguïté (score Mowse 145, couverture de séquence 11%).

Selon une démarche identique, cette analyse SPRi-MALDI-MS/MS a été réitérée avec une autre protéine, la β-lactoglobuline spécifiquement retenue par des anticorps anti-β-lactoglobuline greffés sur la biopuce, puis digérée directement sur la biopuce. Huit peptides issus de la digestion ont été identifiés en MALDI-MS. A partir de deux d'entre eux (m/z 2313.27 et m/z 2707.3), des expériences MS/MS suivies de recherche en banque de données (SwissProt 56.2) ont permis d'identifier les deux séquences peptidiques correspondantes (VYVEELKPTPEGDLEILLQK (SEQ ID NO:5) et VAGTWYSLAMAASDISLLDAQSAPLR (SEQ ID NO:6), respectivement) et par suite la protéine retenue avec un score Mowse de 120 (couverture de séquence 25%).

## Revendications

1. Procédé de fonctionnalisation de la face métallique d'un support d'analyse par résonance des plasmons de surface, ledit procédé comprenant le greffage d'une monocouche autoassemblée de poly(oxy)éthylène directement sur la face métallique dudit support,
dans lequel le métal de la face métallique est de l'or, et
dans lequel le poly(oxy)éthylène (POE) est un composé de formule (I)
A-(CH₂)ₙ-(O-CH₂-CH₂)ₓ-D (I)
dans laquelle
- n est égal à 1 ou 2, en particulier n étant égal à 2;
- x est un nombre entier compris entre 5 à 16, en particulier x étant égal à 8;
- A est un groupement permettant l'ancrage du POE par une liaison covalente sur la surface en or du support, A correspondant en particulier à un groupement -SH ; et
- D est un groupement, éventuellement modifié, permettant la fixation de biomolécules.

2. Procédé selon la revendication 1, le POE étant le O-(2-mercaptoéthyl)-O'-(2-carboxyethyl)heptaéthylène glycol de formule HS-CH₂-CH₂-(O-CH₂-CH₂)₈-COOH.

3. Procédé selon la revendication 1 ou 2, le procédé comprenant la suite d'étapes suivantes :
1) le nettoyage préalable du support, notamment par traitement UV-ozone ;
2) le greffage du POE sur le support ; et
3) éventuellement, la modification du groupe D du POE.

4. Procédé selon la revendication 3, dans lequel le groupe D représente un groupe -COOH qui est modifié à l'étape 3) en groupe ester de succinimide.

5. Procédé selon la revendication 3 ou 4, le greffage étant réalisé en plongeant le support dans un récipient contenant le poly(oxy)éthylène à greffer en solution.

6. Support fonctionnalisé pour l'analyse par résonance des plasmons de surface, comprenant une face métallique sur laquelle est directement greffée une monocouche auto-assemblée de poly(oxy)éthylène,
dans lequel la face métallique est en or, et
dans lequel le poly(oxy)éthylène est un composé de formule (I)
A-(CH₂)ₙ-(O-CH₂-CH₂)ₓ-D (I)
dans laquelle
- n est égal à 1 ou 2;
- x est un nombre entier compris entre 5 à 16;
- A est un groupement permettant l'ancrage du POE par une interaction forte sur la surface en or du support ; et
- D est un groupement, éventuellement modifié, permettant la fixation de biomolécules.

7. Support fonctionnalisé selon la revendication 6, produit par le procédé selon l'une quelconque des revendications 1 à 5.

8. Support fonctionnalisé selon la revendication 6 ou 7, comprenant en outre des molécules « récepteurs » immobilisées sur ledit support via une liaison par l'intermédiaire de poly(oxy)éthylène greffé.

9. Utilisation d'un support fonctionnalisé selon l'une quelconque des revendications 6 à 8, dans une expérience de résonance des plasmons de surface, en particulier dans une expérience de résonance des plasmons de surface par imagerie (SPRi).

10. Utilisation selon la revendication 9, ladite utilisation comprenant la liaison de molécules « récepteurs » à la surface dudit support et l'étude des interactions moléculaires de ces molécules par résonance des plasmons de surface, notamment par SPRi.

11. Utilisation d'un support fonctionnalisé selon l'une quelconque des revendications 6 à 8, dans une expérience de spectrométrie de masse, notamment une spectrométrie de masse réalisée avec une ionisation de type MALDI, afin d'identifier structuralement des analytes qui ont été retenus spécifiquement par des molécules récepteurs liées de façon covalente audit support fonctionnalisé.

12. Utilisation d'un support fonctionnalisé selon la revendication 6 ou 7 pour réaliser deux analyses consécutives :
- une analyse par SPR, en particulier une analyse par résonance des plasmons de surface par imagerie ; puis
- une analyse par spectrométrie de masse, en particulier une analyse de type MALDI, notamment une analyse de type MALDI-TOF.

13. Procédé de couplage d'une analyse par SPR, notamment SPRi, avec une analyse par MALDI MS ou MS/MS, comprenant :
1) l'immobilisation d'une ou plusieurs molécules « récepteurs » sur un support fonctionnalisé selon la revendication 6 ou 7 ; puis
2) la mise en place du support dans un analyseur SPR et l'analyse par SPR des interactions entre la ou les molécules « récepteurs » immobilisées et un échantillon d'analytes ; puis
3) le retrait du support de l'analyseur SPR et sa mise en place dans un spectromètre de masse, et l'analyse structurale par MS des analytes retenus spécifiquement par les molécules « récepteurs » au cours de l'analyse SPR.

14. Procédé de couplage d'une analyse par SPR, notamment SPRi, avec une analyse par MS, notamment MALDI MS, ou MS/MS comprenant :
1) l'immobilisation d'une ou plusieurs molécules « récepteurs » sur un support fonctionnalisé selon la revendication 6 ou 7 ; puis
2) la mise en place du support dans un analyseur SPR et l'analyse par SPR des interactions entre la ou les molécules « récepteurs » immobilisées et un échantillon d'analytes ;
3) la digestion enzymatique localisée in situ des analytes retenus sur les plots des molécules « récepteurs » immobilisées sur le support fonctionnalisé, puis la mise en place du support dans un spectromètre de masse, et l'analyse structurale par MS ou MS/MS des produits de digestion de la ou des analytes présents sur le support fonctionnalisé.

15. Procédé de couplage d'une analyse par SPR, notamment SPRi, avec une analyse par MS, notamment MALDI MS, comprenant :
1) l'immobilisation d'une ou plusieurs molécules «récepteurs» sur un support fonctionnalisé selon la revendication 6 ou 7 ; puis
2) la mise en place du support dans un analyseur SPR et l'analyse par SPR des interactions entre la ou les molécules « récepteurs » immobilisées et un échantillon d'analytes ; puis
3) le retrait du support de l'analyseur SPR et sa mise en place dans un spectromètre de masse, et l'analyse structurale par MS des analytes retenus spécifiquement par les molécules « récepteurs » au cours de l'analyse SPR ; puis
4) la digestion enzymatique localisée *in situ* des analytes retenus sur les plots des molécules « récepteurs » immobilisées sur le support fonctionnalisé, et la mise en place du support dans un spectromètre de masse, et l'analyse structurale par MALDI MS ou MALDI MS/MS des produits de digestion de la ou des molécules présentes sur le support fonctionnalisé.

## Patentansprüche

1. Verfahren zur Funktionalisierung der metallischen Oberfläche eines Oberflächenplasmonenresonanz-Analyseträgers, wobei das Verfahren das Aufpfropfen einer selbstorganisierenden Monoschicht aus Poly(oxy)ethylen unmittelbar auf die metallische Oberfläche des Trägers umfasst,
wobei es sich bei dem Metall der metallischen Oberfläche um Gold handelt, und
wobei es sich bei dem Poly(oxy)ethylen (POE) um eine Verbindung der Formal (I) handelt
A-(CH₂)ₙ-(O-CH₂-CH₂)ₓ-D (I)
wobei
- n gleich 1 oder 2 ist, wobei insbesondere n gleich 2 ist;
- x eine ganze Zahl zwischen 5 und 16 ist, wobei insbesondere x gleich 8 ist;
- A eine Gruppe zur Verankerung des POE mittels kovalenter Bindung auf der Goldoberfläche des Trägers ist, wobei A insbesondere einer Gruppe der Formel -SH entspricht; und
- D eine eventuell modifizierte Gruppe ist, welche die Fixierung von Biomolekülen erlaubt.

2. Verfahren nach Anspruch 1, wobei es sich bei dem POE um O-(2-Mercaptoethyl)-O'-(2-Carboxyethyl)Heptaethylenglykol der Formel HS-CH₂-CH₂-(O-CH₂-CH₂)₈-COOH handelt.

3. Verfahren nach Anspruch 1 oder 2, wobei das Verfahren die folgende Schrittfolge umfasst:
1) das Vorreinigen des Trägers, insbesondere durch Ozon/UV-Behandlung;
2) das Aufpfropfen des POE auf den Träger; und
3) eventuell das Modifizieren der Gruppe D des POE.

4. Verfahren nach Anspruch 3, wobei die Gruppe D eine Gruppe der Formel -COOH darstellt, welche in Schritt 3 zu einer Succinimidester-Gruppe modifiziert wird.

5. Verfahren nach Anspruch 3 oder 4, wobei das Aufpfropfen dadurch erfolgt, dass der Träger in einen Behälter mit dem darin in Lösung befindlichen, aufzupfropfenden Poly(oxy)ethylen getaucht wird.

6. Funktionalisierter Träger für die Oberflächenplasmonenresonanzanalyse, welcher eine metallische Oberfläche umfasst, auf der unmittelbar eine selbstorganisierende Monoschicht aus Poly(oxy)ethylen aufgepfropft ist,
wobei die metallische Oberfläche aus Gold ist, und
wobei es sich bei dem Poly(oxy)ethylen um eine Verbindung der Formel (I) handelt
A-(CH₂)ₙ-(O-CH₂-CH₂)ₓ-D (I)
wobei
- n gleich 1 oder 2 ist;
- x eine ganze Zahl zwischen 5 und 16 ist;
- A eine Gruppe zur Verankerung des POE mittels starker Wechselwirkung auf der Goldoberfläche des Trägers ist; und
- D eine eventuell modifizierte Gruppe ist, welche die Fixierung von Biomolekülen erlaubt.

7. Funktionalisierter Träger nach Anspruch 6, hergestellt durch das Verfahren nach einem der Ansprüche 1 bis 5.

8. Funktionalisierter Träger nach Anspruch 6 oder 7, welcher außerdem "Rezeptor"-Moleküle umfasst, die auf dem Träger mittels einer Bindung über aufgepfropftes Poly(oxy)ethylen immobilisiert sind.

9. Verwendung eines funktionalisierten Trägers nach einem der Ansprüche 6 bis 8 in einem Oberflächenplasmonenresonanz-Experiment, insbesondere einem bildgebenden Oberflächenplasmonenresonanz-Experiment (SPRi).

10. Verwendung nach Anspruch 9, wobei die Verwendung die Bindung von "Rezeptor"-Molekülen an der Oberfläche des Trägers und die Untersuchung der molekularen Wechselwirkungen dieser Moleküle durch Oberflächenplasmonenresonanz, insbesondere durch SPRi, umfasst.

11. Verwendung eines funktionalisierten Trägers nach einem der Ansprüche 6 bis 8 in einem Massenspektrometrie-Experiment, insbesondere einer mit einer MALDI-Ionisierung durchgeführten Massenspektrometrie, um strukturell Analyten zu identifizieren, welche spezifisch von den in kovalenter Weise an den funktionalisierten Träger gebundenen Rezeptormolekülen zurückgehalten worden sind.

12. Verwendung eines funktionalisierten Trägers nach Anspruch 6 oder 7 zur Durchführung zweier aufeinanderfolgender Analysen:
- einer SPR-Analyse, insbesondere einer bildgebenden Oberflächenplasmonenresonanz-Analyse; und darauf folgend
- einer Massenspektrometrie-Analyse, insbesondere einer MALDI-Analyse, vor allem einer MALDI-TOF-Analyse.

13. Verfahren zur Koppelung einer SPR-Analyse, vor allem einer SPRi-Analyse, mit einer MALDI-MS-Analyse oder MALDI-MS/MS-Analyse, umfassend:
1) das Immobilisieren eines oder mehrerer "Rezeptor"-Moleküle auf einem funktionalisierten Träger nach Anspruch 6 oder 7; und darauf folgend
2) das Einbringen des Trägers in ein SPR-Analysegerät und das mittels SPR erfolgende Analysieren der Wechselwirkungen zwischen dem oder den "Rezeptor"-Molekülen und einer Analyten-Probe; und darauf folgend
3) das Entnehmen des Trägers aus dem SPR-Analysegerät und dessen Einbringen in ein Massenspektrometer sowie das mittels MS erfolgende, strukturelle Analysieren der im Verlauf der SPR-Analyse von den "Rezeptor"-Molekülen spezifisch zurückgehaltenen Analyten.

14. Verfahren zur Koppelung einer SPR-Analyse, vor allem einer SPRi-Analyse, mit einer MS-Analyse, vor allem einer MALDI-MS- oder MS/MS-Analyse, umfassend:
1) das Immobilisieren eines oder mehrerer "Rezeptor"-Moleküle auf einem funktionalisierten Träger nach Anspruch 6 oder 7; und darauf folgend
2) das Einbringen des Trägers in ein SPR-Analysegerät und das mittels SPR erfolgende Analysieren der Wechselwirkungen zwischen dem oder den "Rezeptor"-Molekülen und einer Analyten-Probe;
3) das lokalisierte, *in situ* erfolgende enzymatische Verdauen der Analyten, die an den Plots der auf dem funktionalisierten Träger immobilisierten "Rezeptor"-Moleküle zurückgehalten werden, und darauf folgend das Einbringen des Trägers in ein Massenspektrometer und das mittels MS oder MS/MS erfolgende, strukturelle Analysieren der auf dem funktionalisierten Träger vorhandenen Verdauungsprodukte des oder der Analyten.

15. Verfahren zur Koppelung einer SPR-Analyse, vor allem einer SPRi-Analyse, mit einer MS-Analyse, vor allem einer MALDI-MS-Analyse, umfassend:
1) das Immobilisieren eines oder mehrerer "Rezeptor"-Moleküle auf einem funktionalisierten Träger nach Anspruch 6 oder 7; und darauf folgend
2) das Einbringen des Trägers in ein SPR-Analysegerät und das mittels SPR erfolgende Analysieren der Wechselwirkungen zwischen dem oder den immobilisierten "Rezeptor"-Molekülen und einer Analyten-Probe; und darauf folgend
3) das Entnehmen des Trägers aus dem SPR-Analysegerät und dessen Einbringen in ein Massenspektrometer sowie das mittels MS erfolgende, strukturelle Analysieren der im Verlauf der SPR-Analyse von den "Rezeptor"-Molekülen spezifisch zurückgehaltenen Analyten; und darauf folgend
4) das lokalisierte, *in situ* erfolgende enzymatische Verdauen der Analyten, die an den Plots der auf dem funktionalisierten Träger immobilisierten "Rezeptor"-Moleküle zurückgehalten werden, und das Einbringen des Trägers in ein Massenspektrometer und das mittels MALDI-MS oder MALDI-MS/MS erfolgende, strukturelle Analysieren der auf dem funktionalisierten Träger vorhandenen Verdauungsprodukte des oder der Moleküle.

## Claims

1. A process for functionalizing the metal face of a support for analysis by surface plasmon resonance, said process comprising grafting a self-assembled monolayer of poly(oxy)ethylene directly onto the metal face of said support,
wherein the metal of the metal face is gold, and
wherein the poly(oxy)ethylene (POE) is a compound of formula (I)
A-(CH₂)ₙ-(O-CH₂-CH₂)ₓ-D (I)
wherein
- n is equal to 1 or 2, in particular n being equal to 2;
- x is an integer comprised between 5 and 16, in particular x being equal to 8;
- A is a group for anchoring the POE onto the gold surface of the support by a covalent bond, A corresponding in particular to an -SH group; and
- D is an optionally modified group for the binding of biomolecules.

2. The process according to claim 1, the POE being O-(2-mercaptoethyl)-O'-(2-carboxyethyl)heptaethylene glycol of formula HS-CH₂-CH₂-(O-CH₂-CH₂)₈-COOH.

3. The process according to claim 1 or 2, the process comprising the following sequence of steps:
1) preliminary cleaning of the support, in particular by UV-ozone treatment;
2) grafting of the POE onto the support; and
3) optionally, modification of group D of the POE.

4. The process according to claim 3, wherein group D represents a -COOH group which is modified in step 3) so as to give a succinimide ester group.

5. The process according to claim 3 or 4, the grafting being carried out by immersing the support in a vessel containing the poly(oxy)ethylene to be grafted in solution.

6. A functionalized support for analysis by surface plasmon resonance, comprising a metal face onto which a self-assembled monolayer of poly(oxy)ethylene is directly grafted,
wherein the metal face is made of gold, and
wherein the poly(oxy)ethylene is a compound of formula (I)
A-(CH₂)ₙ-(O-CH₂-CH₂)ₓ-D (I)
wherein
- n is equal to 1 or 2;
- x is an integer comprised between 5 and 16;
- A is a group for anchoring the POE onto the gold surface of the support by a strong interaction; and
- D is an optionally modified group for the binding of biomolecules.

7. The functionalized support according to claim 6, produced by the process according to any one of claims 1 to 5.

8. The functionalized support according to claim 6 or 7, further comprising "receptor" molecules immobilized on said support via a bond by means of grafted poly(oxy)ethylene.

9. Use of a functionalized support according to any one of claims 6 to 8, in a surface plasmon resonance experiment, in particular in a surface plasmon resonance imaging (SPRi) experiment.

10. Use according to claim 9, said use comprising linking "receptor" molecules to the surface of said support and studying the molecular interactions of these molecules by surface plasmon resonance, in particular by SPRi.

11. Use of a functionalized support according to any one of claims 6 to 8, in a mass spectrometry experiment, in particular mass spectrometry carried out with MALDI-type ionization, in order to structurally identify analytes which have been specifically retained by receptor molecules covalently linked to said functionalized support.

12. Use of a functionalized support according to claim 6 or 7 for carrying out two consecutive analyses:
- an analysis by SPR, in particular an analysis by surface plasmon resonance imaging; then
- an analysis by mass spectrometry, in particular a MALDI-type analysis, in particular a MALDI-TOF-type analysis.

13. A process for coupling an analysis by SPR, in particular SPRi, with an analysis by MALDI MS or MS/MS, comprising:
1) immobilizing one or more "receptor" molecules on a functionalized support according to claim 6 or 7; then
2) placing the support in an SPR analyzer and analyzing by SPR the interactions between the immobilized "receptor" molecule(s) and an analyte sample; then
3) removing the support from the SPR analyzer and placing said support in a mass spectrometer, and structurally analyzing by MS the analytes specifically retained by the "receptor" molecules during the SPR analysis.

14. A process for coupling an analysis by SPR, in particular SPRi, with an analysis by MS, in particular MALDI MS, or MS/MS comprising:
1) immobilizing one or more "receptor" molecules on a functionalized support according to claim 6 or 7; then
2) placing the support in an SPR analyzer and analyzing by SPR the interactions between the immobilized "receptor" molecule(s) and an analyte sample;
3) *in situ* localized enzyme digesting the analytes retained on the spots of "receptor" molecules immobilized on the functionalized support, then placing the support in a mass spectrometer, and structurally analyzing by MS or MS/MS the digestion products of the analyte(s) present on the functionalized support.

15. A process for coupling an analysis by SPR, in particular SPRi, with an analysis by MS, in particular MALDI MS, comprising:
1) immobilizing one or more "receptor" molecules on a functionalized support according to claim 6 or 7; then
2) placing the support in an SPR analyzer and analyzing by SPR the interactions between the immobilized "receptor" molecule(s) and an analyte sample; then
3) removing the support from the SPR analyzer and placing said support in a mass spectrometer, and structurally analyzing by MS the analytes specifically retained by the "receptor" molecules during the SPR analysis; then
4) *in situ* localized enzyme digesting the analytes retained on the spots of "receptor" molecules immobilized on the functionalized support, and placing the support in a mass spectrometer, and structurally analyzing by MALDI MS or MALDI MS/MS the digestion products of the molecule(s) present on the functionalized support.


### SEQUENCE LISTING

<110> GENOPTICS CNRS UNIVERSITE D'EVRY VAL D'ESSONNE
<120> BIOPUCES FONCTIONNALISEES POUR LE COUPLAGE SPR-MS
<130> B626PC00
<160> 6
<170> PatentIn version 3.3
<210> 1
   <211> 386
   <212> PRT
   <213> Gallus gallus
<400> 1
   Met Gly Ser Ile Gly Ala Ala Ser Met Glu Phe Cys Phe Asp Val Phe
   1 5 10 15
   Lys Glu Leu Lys Val His His Ala Asn Glu Asn Ile Phe Tyr Cys Pro
   20 25 30
   Ile Ala Ile Met Ser Ala Leu Ala Met Val Tyr Leu Gly Ala Lys Asp
   35 40 45
   Ser Thr Arg Thr Gln Ile Asn Lys Val Val Arg Phe Asp Lys Leu Pro
   50 55 60
   Gly Phe Gly Asp Ser Ile Glu Ala Gln Cys Gly Thr Ser Val Asn Val
   65 70 75 80
   His Ser Ser Leu Arg Asp Ile Leu Asn Gln Ile Thr Lys Pro Asn Asp
   85 90 95
   Val Tyr Ser Phe Ser Leu Ala Ser Arg Leu Tyr Ala Glu Glu Arg Tyr
   100 105 110
   Pro Ile Leu Pro Glu Tyr Leu Gln Cys Val Lys Glu Leu Tyr Arg Gly
   115 120 125
   Gly Leu Glu Pro Ile Asn Phe Gln Thr Ala Ala Asp Gln Ala Arg Glu
   130 135 140
   Leu Ile Asn Ser Trp Val Glu Ser Gln Thr Asn Gly Ile Ile Arg Asn
   145 150 155 160
   Val Leu Gln Pro Ser Ser Val Asp Ser Gln Thr Ala Met Val Leu Val
   165 170 175
   Asn Ala Ile Val Phe Lys Gly Leu Trp Glu Lys Ala Phe Lys Asp Glu
   180 185 190
   Asp Thr Gln Ala Met Pro Phe Arg Val Thr Glu Gln Glu Ser Lys Pro
                   195                 200                 205
   Val Gln Met Met Tyr Gln Ile Gly Leu Phe Arg Val Ala Ser Met Ala
       210                 215                 220
   Ser Glu Lys Met Lys Ile Leu Glu Leu Pro Phe Ala Ser Gly Thr Met
   225                 230                 235                 240
   Ser Met Leu Val Leu Leu Pro Asp Glu Val Ser Gly Leu Glu Gln Leu
                   245                 250                 255
   Glu Ser Ile Ile Asn Phe Glu Lys Leu Thr Glu Trp Thr Ser Ser Asn
               260                 265                 270
   Val Met Glu Glu Arg Lys Ile Lys Val Tyr Leu Pro Arg Met Lys Met
           275                 280                 285
   Glu Glu Lys Tyr Asn Leu Thr Ser Val Leu Met Ala Met Gly Ile Thr
       290                 295                 300
   Asp Val Phe Ser Ser Ser Ala Asn Leu Ser Gly Ile Ser Ser Ala Glu
   305                 310                 315                 320
   Ser Leu Lys Ile Ser Gln Ala Val His Ala Ala His Ala Glu Ile Asn
                   325                 330                 335
   Glu Ala Gly Arg Glu Val Val Gly Ser Ala Glu Ala Gly Val Asp Ala
               340                 345                 350
   Ala Ser Val Ser Glu Glu Phe Arg Ala Asp His Pro Phe Leu Phe Cys
           355                 360                 365
   Ile Lys His Ile Ala Thr Asn Ala Val Leu Phe Phe Gly Arg Cys Val
       370                 375                 380
   Ser Pro
   385
<210> 2
   <211> 13
   <212> PRT
   <213> Gallus gallus
<400> 2
   Ala Phe Lys Asp Glu Asp Thr Gln Ala Met Pro Phe Arg
   1               5                   10
<210> 3
   <211> 16
   <212> PRT
   <213> Gallus gallus
<400> 3
   Gly Gly Leu Glu Pro Ile Asn Phe Gln Thr Ala Ala Asp Gln Ala Arg
   1               5                   10                  15
<210> 4
   <211> 17
   <212> PRT
   <213> Gallus gallus
<400> 4
   Ile Ser Gln Ala Val His Ala Ala His Ala Glu Ile Asn Glu Ala Gly
   1               5                   10                  15
   Arg
<210> 5
   <211> 20
   <212> PRT
   <213> Bos taurus
<400> 5
   Val Tyr Val Glu Glu Leu Lys Pro Thr Pro Glu Gly Asp Leu Glu Ile
   1               5                   10                  15
   Leu Leu Gln Lys
               20
<210> 6
   <211> 26
   <212> PRT
   <213> Bos taurus
<400> 6
   Val Ala Gly Thr Trp Tyr Ser Leu Ala Met Ala Ala Ser Asp Ile Ser
   1               5                   10                  15
   Leu Leu Asp Ala Gln Ser Ala Pro Leu Arg
               20                  25